Europäisches Patentamt

European Patent Office

Office européen des brevets

(11) Publication number: **0 212 444 B1**

## EUROPEAN PATENT SPECIFICATION

(45) Date of publication of patent specification: **25.03.92**  (51) Int. Cl.5: **C12N 15/63**

(21) Application number: **86110852.0**

(22) Date of filing: **06.08.86**

(54) Process of producing highly transformable cells and cells produced thereby.

(30) Priority: **08.08.85 US 763825**

(43) Date of publication of application:
**04.03.87 Bulletin  87/10**

(45) Publication of the grant of the patent:
**25.03.92 Bulletin  92/13**

(84) Designated Contracting States:
**AT BE CH DE FR GB IT LI LU NL SE**

(56) References cited:

**S.P. COLOWICK and N.O. KAPLAN: "Methods in Enzymology", vol. 68: "Recombinant DNA", 1979, pages 326-331, Academic Press, New York, US; D.A. MORRISON: "Transformation and preservation of competent bacterial cells by freezing"**

**JOURNAL OF MOLECULAR BIOLOGY, vol. 166, 1983, pages 557-580, Academic Press Inc., London, GB; D. HANAHAN: "Studies on transformation of Escherichia Coli with plasmids"**

(73) Proprietor: **LIFE TECHNOLOGIES INC.**
**8717 Grovemont Circle, P.O. Box 6009**
**Gaithersburg, MD 20877(US)**

(72) Inventor: **Jessee, Joel A.**
**21 Nancy Place**
**Gaithersburg Maryland 20877(US)**
Inventor: **Bloom, Fredric R.**
**9454 Horizon Run Road**
**Gaithersburg Maryland 20879(US)**

(74) Representative: **Vossius & Partner**
**Siebertstrasse 4 P.O. Box 86 07 67**
**W-8000 München 86(DE)**

**Description**

BACKGROUND OF THE INVENTION

This invention relates to a process for producing cells of improved competency that are tranformable ("competency" referring to the ability of cells to take up and establish exogenous DNA), and to such competent cells. This aspect of the invention relates particularly to cells of Escherichia coli having competency for plasmid DNA. The invention further relates to such a process and to cells produced by the process, which can be repeatedly frozen and thawed without significantly loss in transformability.

The methods used for the insertion of exogenous DNA sequences and genes from a variety of organisms into Escherichia coli have been described in U.S. Patent No. 4,237,224 (Cohen and Boyer, 1980). That patent describes the preparation of hybrid plasmid molecules, the insertion of these hybrid plasmid molecules into cells of E. coli, and the replication and amplification of these recombinant plasmids in the transformed cells. The extensive use of an interest in recombinant DNA technology has resulted in a great need for E. coli cells which are capable of taking up and establishing recombinant plasmid molecules, since competent E. coli have become important to the process of introducing and amplifying exogenous genes or sequences.

A number of procedures exist for the preparation of competent E. coli cells and the introduction of DNA into these cells. For example Mandel and Higa (1970, Journal of Molecular Biology 53:159) describe a procedure whereby bacteriophage DNA is combined with E. coli cells in the presence of 50 mM $Ca^{++}$ at 0°C followed by a brief heat pulse at 37-42°C. This method has been extended to the uptake of chromosomal DNA (Cosloy and Oishi 1973, Proceedings of the National Academy of Science 70:84) and plasmid DNA (Cohen et al 1972, PNAS 69:2110). A summary of the factors influencing the efficiency of transformation is given in Hanahan (1983, JMB 166:557). These factors include the addition of other cations such as Mg, Mn, or Rb to the Ca-treated cells as well as the prolonged incubation of the cells in $CaCl_2$. The efficiency of transformation of E. coli cells is substantially enhanced by the method described by Hanahan (1983, JMB, hereinafter referred to as "Hanahan (1983)"). The E. coli cells are grown at 37°C in the presence of 20 mM Mg. Plasmid DNA is combined with the cells at 0°C in the presence of Mn, Ca, Rb or K, dimethylsulfoxide (DMSO), dithiothreitol (DTT) and hexamine cobalt chloride.

Several E. coli strains prepared by the latter method have transformation efficiencies of from 1 to 5 x $10^8$ transformants/ug plasmid DNA. Generally frozen competent cells have transformation efficiencies of about 1 x $10^8$ transformants/ug plasmid DNA. These competent cells can be stored frozen at-70°C for several months without significant loss of transformation efficiency. However, the foregoing frozen cells cannot be thawed and refrozen without significant loss of transformation efficiency.

Summary of the Invention

It is an object of the present invention to provide cells of improved competency.

It is a further object of the invention to provide highly competent cells that have high transformation efficiencies.

Another object of the invention is the provision of competent, highly transformable cells that can repeatedly be frozen and thawed without significantly reducing their transformability.

An additional object is to provide highly competent, highly transformable E. coli cells.

Yet another object is to provide a process for making cells of improved competency.

It is a further object of the invention to provide a process for producing highly competent cells that are transformable.

Another object is the provision of a process for producing competent cells that are highly transformable, and that can repeatedly be frozen and thawed without significantly reductions in competency.

It is still a further object to provide a process for producing highly transformable E. coli of improved competency.

Other objects will be apparent from the description to follow and from the appended claims

The present invention in its preferred forms provides processes for the preparation of frozen competent E. coli cells having transformation efficiencies greater than 5 x $10^8$ transformants per ug plasmid DNA, generally greater than $10^9$ transformants/ug plasmid DNA. These cells have the useful property of being capable of being repeatedly frozen and thawed without substantial loss of transformation efficiency.

Detailed Description of the Preferred Embodiments

The processes described below involve the growth of E. coli cells in a growth conducive medium such as a complex medium containing Mg at temperatures of less than 37°C, and generally between 18-32°C for a time period of between 1-20 hours, or until the optical density ("O.D.") of the culture at 550 nm reaches 0.5-1.0, washing the cells in a buffer containing 10 mM potassium acetate, 100 mM KCl, 45 MnCl$_2$•4H$_2$O, 10 mM CaCl$_2$.2H$_2$O, 3 mM hexamine cobalt chloride, and 10% redistilled glycerol according to the procedure of Hanahan (1983), incubating cells with DMSO and freezing the cells in a dry ice-ethanol bath and storing the cells at -70°C. Growth of the cells at temperatures between 18°C and 32°C as well as the subsequent freezing step are both essential to the process of preparing highly competent E. coli cells with the property of being repeatedly frozen and thawed without substantial loss of transformation efficiency. The E. coli strains prepared according to the method described below are useful for the preparation of DNA gene banks as well as for the routine subcloning of genes.

The preferred procedure for preparing competent cells of E. coli is based on the method of Hanahan (1983) with the following modifications: (1) the cells are grown at temperatures less than 37°C, and generally between 18°C to 32°C with the preferred growth temperature depending on the strain of E. coli; (2) sodium chloride is present in the growth medium for some E. coli strains; (3) sodium succinate is present in the fermentation medium, and (4) for large volumes of cells, 250 ml Corning centrifuge bottles are used for the rapid resuspension of cell pellets. Reference is made to Hanahan (1983) for details of the procedure described below which are not expressly stated herein.

Materials and Methods

The comments set forth below include various conclusions and recommendations based on various techniques practiced according to the invention.

Media

S.O.B., S.O.C., and LM agar plates are defined and described by Hanahan (1983). S.O.B. with 0.5% sodium succinate is used in fermentation broths. S.O.B. and LM agar plates with sodium chloride are prepared by adding sodium chloride after the medium is autoclaved. YET ampicillin 100 agar plates are 10 g/l BactoTrytone, 5 g/l Yeast Extract, 5 g/l NaCl, and 100 ug/ml Ampicillin and 15 g/l Bactoagar.

Buffers

F.S.B. is made as defined and described by Hanahan (1983). F.S.B. buffer containing 5% sucrose (w/v) is used when making strains DH1 and DH5 competent. S.O.B./glycerol solution (60/40 v/v) is prepared as described by Hanahan (1983).

DNA

Monomer pBR322 plasmid DNA is used to determine the transformation efficiency of the cells. Plasmid DNA is prepared from Escherichia coli strain HB101/pBR322 by the use of CsCl$_2$ density gradients.

Transformation

The procedure used to determine the transformation efficiency of the competent cells is described by Hanahan (1983). Modifications of this procedure such as using 0.1 ml rather than 0.2 ml of competent cells have also been used to determine transformation efficiency of the cells.

Freeze Thaw Protocol

A 550 ul aliquot of frozen competent cells is placed on ice until the cells have thawed. The appropriate amount of cells is removed. The cells are then frozen in a dry ice ethanol ("ETOH") bath for five minutes and placed back in a -70°C freezer until the cells are used again.

E. coli Strains

RR1      F$^-$ hsdS20 (r$_B^-$ ,m $_B^-$) supE44 araI4 galK2
           lacYl proA2 leu rpsL20 xyl-5 mtl-1

3

HB101    $F^-$ hsdS20 ($r_B^-$ ,m $\overline{B}$) supE44 araI4 galK2
         lacYI proA2 leu rpsL20 xyl-5 $m^+$ mtl-1 $+$1-1 recAI3

DH1    $F^-$ endA1 hsdR17 ($r_K^-$ , $m_K^+$ ) supE44 thi-1
         recAI gyrA96 relAI

DH5    $F^-$ endAI hsdR17 ($r_K^-$ , $m_K^+$ ) supE44 thi-1
         recAI gyrA96 relAI

## Reagents and Equipment

All reagents should be of the highest quality available. The water used to make solutions should be of the purest quality Milli Q water (Milli Q Corporation) is recommended (Hanahan 1983). All glassware should be extremely clean. All glassware should be rinsed with distilled water and culture flasks should be autoclaved with distilled water in them prior to use.

## General Comments Concerning the Examples

### Preparation of Master Seeds

Strains of Escherichia coli were stored as -70°C glycerol stocks. The procedure used to make these master seeds is described by Hanahan (1983). A pure source of the bacterial strain (such as an agar stab, a -70°C glycerol stock or colonies on an agar plate) was streaked on LM agar plates and the plates were incubated at 37°C for a period of time that resulted in colonies of 1-2 mm in diameter for generally 10 to 16 hours. Two colonies from the LM agar plates were inoculated into 1-2 mls of S.O.B. medium and vortexed. This inoculum was used to inoculate 30-50 ml of S.O.B. in a 500 ml nonbaffled flask. This culture was grown at 37°C, 200-300 RPM to an $O.D._{550}$ of 0.5-0.7. The speed of shaking should be rapid enough to give good aeration but no foaming. When the culture reached the appropriate optical density, an aliquot was removed and added to an equal volume of 60% S.O.B. and 40% glycerol (v/v) in a sterile tube. The cells were left on ice for 10 minutes. The cells were then aliquoted into sterile chilled 4°C Nunc tubes and frozen at -70°C in a dry ice ETOH bath for five minutes. Master seeds were stored at -70°C. These master seeds found to be stable for over a year.

### Preparation of Primary Seeds

Primary seeds are used to inoculate cultures that will serve as the inoculum for a fermentation. Primary seeds will also generate colonies for inoculum of a shake flask. A master seed was removed from a -70°C freezer and put on dry ice so as not to thaw. The surface of the frozen seed was scraped and frozen cells were placed on the surface of an LM agar plate or an LM NaCl agar plate. The optimal salt concentration, generally between 0-0.3 M was determined for each E. coli strain. The pool of cells was streaked to isolate single colonies. The plates were incubated at the preferred growth temperature, generally between 18°C and 32°C. The growth temperature for the culture used to make the primary seed is generally the temperature at which the cells will be made competent. However the primary seed can be made from a culture grown at a higher growth temperature and that primary seed can be used to prepare competent cells from a culture grown at a lower temperature.

The colonies that appeared on the LM agar plates or LM NaCl agar plates were used to inoculate a 500 ml nonbaffled shake flask with 30 to 50 ml of S.O.B. or S.O.B. NaCl (depending on the strain of E. coli). The shake flask culture was grown at the appropriate temperature to an $O.D._{550}$ equal to 0.7. From this culture an aliquot was removed and mixed with an equal volume of 60% S.O.B./40% glycerol. Cells were left on ice for 10 minutes and then aliquoted into sterile chilled 4°C Nunc tubes (0.5-1.0 ml/tube). The cells were then frozen in a dry ice/ETOH bath. Primary seeds and master seeds were stored at -70°C, and it has been determined that they should be remade on a yearly basis.

### Shake Flask Production of Frozen Competent Cells

A primary seed stock is used as a source of the inoculum for cultures grown in shake flasks. A primary seed was removed from a -70°C freezer and placed on dry ice. The surface of the seed was scraped and the cells placed on an LM agar plate containing an appropriate concentration of sodium chloride. The plate was incubated at the appropriate temperature generally between 18-32°C according to the E. coli strain used. Generally the growth temperature and sodium chloride concentration are the same as used to make the primary seed. Colonies were allowed to reach a size of 1-2 mm and the colonies were picked off the plate into 1 to 2 mls of S.O.B. medium. This inoculum was used to inoculate shake flasks with a large surface to volume ratio. A good rate of shaking was found to be 250-275 rpm, the object being to obtain good mixing with as little foaming as possible. A Fernbach flask (2.8 l) containing 200 ml of S.O.B. with the appropriate amount of added sodium chloride was inoculated with an appropriate amount of the inoculum (depending on the temperature of growth). Generally cells grown between 18°C-32°C were found to have a doubling time which allowed for an overnight growth. The object was to obtain a culture that has an O.D.$_{550}$ of 0.5 ± 0.2. When the optimal O.D.$_{550nm}$ was reached, the culture was poured into centrifuge bottles that could be spun at 4000 rpm and still allow for a rapid resuspension of cells. Conical centrifuge tubes made of polypropylene are preferred for this purpose. The cells were centrifuged 4000 rpm for 10 minutes at 4°C and the cell supernate was poured off. The bottles were tapped to remove as much liquid as possible. Chilled 4°C F.S.B. buffer was added to each cell pellet to 1/3 of the original volume, and the cells were brought into solution by tapping the bottle. After resuspension, the cells were left on ice for 15 minutes and then centrifuged at 4000 rpm at 4°C for 10 minutes. The cell pellet was then resuspended in 1/12.5 of the original cell supernate volume using chilled 4°C F.S.B. buffer. DMSO was added to 3.5% (v/v) and the cells were left on ice for five minutes. Another 3.5% portion of DMSO was added to the cells, and the cells were mixed and set on ice for 10 minutes. The total time for exposure of the cells to DMSO was approximately fifteen minutes. The cells were frozen in a dry ice/ETOH bath (-70°C) for five minutes. Aliquots of 25 mls were frozen in 50 ml polypropylene tubes, while smaller aliquots were frozen in 0.5 mls aliquots. It was found that the aliquots should be completely frozen in 5 minutes.

Fermentation Growths for Production of Frozen Competent Cells

Fermentation growths require a liquid inoculum for the fermenter. The seed culture for the fermenter is originated from a primary seed stock. The liquid culture generally is started the day before the fermenter is inoculated for cells grown at 22-23°C, or the liquid culture is started on the day of the fermentation for cells grown at 30°-32°C. The liquid seed culture is grown using the same conditions as the culture used to make the primary seed. The liquid seed was grown to an O.D.$_{550}$ of approximately 0.1 and the liquid seed was used to inoculate a fermenter. The volume of seed culture used to inoculate the fermenter was varied from strain to strain, and had to be calibrated so that the fermentation culture reached an O.D.$_{550}$ of 1.0 at the appropriate time. The medium used in the fermentation was S.O.B. containing 5.0 g/l sodium succinate and a sodium chloride concentration determined as discussed earlier that gave the highest transformation frequency for the E. coli strain. It was found that the culture should be agitated at 300-400 rpm and aerated at 1.0 - 2.0 vvm. The fermentation medium should be autoclaved for 10 minutes at 121°C and then cooled to the temperature of growth. Sodium chloride, magnesium sulfate, and magnesium chloride were added after the medium had been autoclaved and cooled. The fermentation was allowed to proceed to a final O.D.$_{550}$ of 0.1. The culture was then placed in 250 ml centrifuge bottles that allowed for easy resuspension of the cells. Cells were set on ice for five minutes and centrifuged for 10 minutes at 4°C and 4000 rpm. The cell supernate was poured off and the cells were resuspended in 16/25 of original culture volume using chilled 4°C F.S.B. buffer. The cells were set on ice for 15 minutes. DMSO was added to 3.5%, mixed well, and the cells were set on ice for 10 minutes. DMSO was added again to 3.5% and the cells were left on ice for five minutes. It was found that the process should be completely as rapidly as possible, i.e. from 1 to 1.25 hours in order to process 1500 ml of a culture. It was concluded that the cells should then be frozen in a dry ice/ETOH bath as 25 ml bulk portions or as small aliquots. Freezing should be rapid enough to allow for complete freezing in five minutes for the 25 ml bulk portions.

Example 1

A primary seed of Escherichia coli strain RR1 prepared from a culture grown at 23°C in S.O.B. and 0.3 M NaCl was streaked on LM 0.3 M NaCl agar plates. The plates were incubated at 23°C for approximately 36 hours or until colonies of 1-2 mm in size were reached. Twenty colonies from these plates were picked into 2.0 ml S.O.B. with a sterile wooden applicator stick. The inoculum was vortexed and 2 mls were added to 200 mls of S.O.B. and 0.3 M NaCl in a 2.8 liter nonbaffled Fernbach flask. The flask was shaken at 275 rpm at 22°C-23°C for about 15 to 20 hours until an O.D.$_{550}$ of 0.5 was reached. Two fifty ml aliquots were placed in chilled 4°C 50 ml Corning tubes. The Corning tubes were placed on ice for 5-10 minutes. The tubes were spun at 2000 rpm, 4°C for 10 minutes. The supernate was poured off, 16.7 ml of chilled 4°C F.S.B. were added to resuspend the cells and the tubes were placed on ice for 15 minutes. The tubes were then spun as above and the cells resuspended in 4 ml of chilled 4°C F.S.B. buffer. 0.140 ml of DMSO were added to the cells, mixed well and the tubes were set on ice for 10 minutes. 0.140 ml DMSO was added again and the tubes were left on ice for five minutes. Two 0.5 mls aliquots of cells were frozen in a dry ice/ETOH bath. The remaining nonfrozen cells (200μl)were used for a transformation assay with 50 pg pBR322 plasmid DNA and the transformed cells were plated on YET Ampicillin 100 agar plates. The frozen cells were thawed and 200 ul of the cells were transformed as above with 50 pg pBR322 plasmid DNA. All plates were incubated at 37°C for 24 hours. The results are given in Table 1 (lines 1 and 2).

A primary seed of strain RR1 was also used as a source of bacteria for growing the cells at 37°C and making them competent. The primary seed was streaked on LM + 0.3 M NaCl agar plates and the plates were incubated at 37°C for approximately 16 hours. Twenty colonies were picked into 2 ml of S.O.B. and vortexed. The 2 ml was used to inoculate 200 ml S.O.B. and 0.3 M NaCl in a 2.8 liter nonbaffled Fernbach flask. The cells were grown at 37°C, 275 rpm to an O.D.$_{550}$ of 0.5 and made competent by the same procedure discussed above. The frozen cells were thawed and transformed with 50 pg pBR322 plasmid DNA. Cells that were not frozen were also transformed with 50 pg pBR322 plasmid DNA. Transformants are selected using YET Ampicillin 100 agar plates. The results are given in Table 1 (lines 3,4).

Table 1

| The Effect of Growth Temperature and Freezing on the Transformation Efficiency of E. coli Strain RR1 | | |
|---|---|---|
| Growth Temperature | Cells Frozen | Ampicillin Resistant Transformants/ug pBR322 DNA |
| 23°C | Yes | $4.2 \times 10^8$ - $3 \times 10^9$ |
| 23°C | No | $4.0 \times 10^7$ - $1 \times 10^8$ |
| 37°C | Yes | $3.0 \times 10^7$ - $5 \times 10^7$ |
| 37°C | No | $2.4 \times 10^7$ - $5 \times 10^7$ |

By comparing lines 1 and 2, it is concluded that freezing cells grown at 23°C results in an increased transformation efficiency compared to cells grown at 23°C and not frozen. In addition RR1 cells grown at 37° either frozen or not frozen (lines 3 and 4) are less competent than cells grown at 23°C and frozen. Therefore high transformation efficiencies are achieved by growth of the RR1 cells at 23°C and the subsequent freezing of the cells.

RR1 cells grown at either 23°C or 37°C were frozen and thawed according to the procedure in the materials and methods section. 200 ul samples of the cells were transformed with 50 pg pBR322 plasmid DNA. The transformed cells were plated on YET ampicillin 100 agar plates. The results are presented in Table 2.

Table 2

| Freeze Thaw Cycle vs. Transformation Efficiency for E. coli Strain RR1 Grown at 23°C and 37°C | | |
|---|---|---|
| Freeze/Thaw Cycle | Ampicillin Resistant Transformants/ug pBR322 DNA | |
| | Cells grown at 23°C | Cells grown at 37°C |
| 1 | $8.0 \times 10^8$ | $4.6 \times 10^7$ |
| 2 | $6.7 \times 10^8$ | $2.0 \times 10^6$ |
| 3 | $8.3 \times 10^8$ | $<1 \times 10^6$ |
| 4 | $5.7 \times 10^8$ | $<1 \times 10^6$ |
| 5 | $7.4 \times 10^8$ | $<1 \times 10^6$ |

Cells grown at low temperatures are capable of undergoing repeated freeze thaw cycles without significant loss of transformation efficiency whereas cells grown at 37°C cannot undergo the freeze thaw cycles without loss of transformation efficiency.

Example 2

A primary seed of E. coli strain DH5 derived from a culture grown at 30°C with no sodium chloride was removed from the freezer and allowed to thaw at room temperature. The primary seed was diluted to 1/10 in S.O.B./ and 0.1 - 0.3 ml was added to 50 mls of S.O.B. in a 500 ml nonbaffled shake flask. The flask was inoculated at about 9:00 A.M. on the day of the fermentation. The culture was allowed to grown at 30°C to an $O.D._{550}$ equal to 0.1 and 0.2 ml of a 1/10 dilution was used to inoculate a 10 liter fermenter containing S.O.B. and 0.5% sodium succinate pH 7.0. The fermenter was stirred at 300 RPM, and aerated at 1 vvm. The fermenter was inoculated such that it reached an $O.D._{550}$ of 1.0, on the following day. On the following day when the fermenter reached an $O.D._{550}$ equal to 1.0, the cells were removed into sterile, chilled 4°C 250 ml Corning bottles. Twelve bottles were filled and placed on ice for five minutes. The Corning bottles were spun 4000 rpm, 4°C for 10 minutes. The cell supernate was poured off, excess liquid was drained and 40 mls of chilled 4°C FSB buffer were added. The bottles were left on ice for five minutes. 1.4 mls of DMSO was added, the cells were mixed and then left on ice for 10 minutes. Another 1.4 mls portion of DMSO was added and the bottled were set on ice for five minutes. The twelve 40 ml aliquots of cells were mixed, aliquoted into 25 ml portions in 50 ml polypropylene tubes, and the tubes were frozen at -70°C for five minutes. A convex knob of F.S.B. appeared at the surface of the 25 ml aliquots. The results of a transformation assay using 200 ul of cells and 50 pg of pBR322 plasmid DNA indicated that the cells were competent at $1.3 \times 10^9$ transformants/ug pBR322 plasmid DNA.

Example 3

A primary seed of E. coli strain RR1 was streaked on L14 agar plates and the plates were incubated at 37°C for 16 hours. Twenty colonies were picked into 2 mls of S.O.B. and 0.3 M NaCl. The cells were inoculated into 200 mls of S.O.B. and 0.3 M NaCl in a 2.8 liter nonbaffled Fernbach flask. The cells were grown at 37°C and shaken at 275 rpm to a final $O.D._{550}$ of 0.25. The flask was placed in an ice bath and cooled to approximately 23°C. A 30 ml sample of cells was removed and the cells were made competent according to the procedure given in Example 1. The flask was then shaken at 23°C and at 275 rpm for two hours. At 30 minute intervals, 30 ml samples were removed and the cells made competent according to the procedure given in Example 1. 200 ul portions of each sample were transformed with 50 pg PBR322 plasmid DNA. Transformants were selected on YET Ampicillin 100 agar plates and counted after the plates had been incubated at 37°C for 24 hours. The results are presented in Table 3.

Table 3

| Transformation Efficiency: Effect of 37°C to 23°C Temperature Shift on E. coli Strain RR1 | |
| --- | --- |
| Time After Temperature Shift to 23°C (minutes) | Ampicillin Resistant Transformants/ug pBR322 Plasmid DNA |
| 0 | $7 \times 10^6$ |
| 30 | $1.1 \times 10^7$ |
| 60 | $4.0 \times 10^7$ |
| 90 | $9.9 \times 10^7$ |
| 120 | $4.6 \times 10^8$ |

The results of this experiment indicate that shifting strain RR1 from 37°C to 23°C for two hours results in a 60 fold increase in the efficiency of transformation compared to a sample which does not undergo a temperature shift.

**Claims**

1. A process for producing transformable, competent E.coli cells, said process comprising the following steps:
   growing the cells in a growth conducive medium at a temperature in the range of 18°C to 32°C;
   harvesting the cells;
   rapidly resuspending the cells in an appropriate buffer;
   and freezing the cells at approximately -70°C.

2. The process according to claim 1 wherein the E. coli cells have competency for plasmid DNA.

3. The process according to any one of claims 1 or 2 wherein the E. coli cells are at least one of the following strains: RR1, HB101, DH1, DH5.

4. The process according to any one of claims 1 to 3 wherein the growth conducive medium is a complex medium containing Mg.

5. The process according to any one of claims 1 to 4 wherein the step of growing the cells includes growing the cells for a period within a range of from 1 to 20 hours.

6. The process according to any one of claims 1 to 5 wherein the step of growing the cells includes growing the cells until the optical density at 550 nm reaches the range of 0.5 to 1.0.

7. The process according to any one of claims 1 to 6, further including the step of washing the cells in a buffer prior to the freezing step.

8. The process according to any one of claims 1 to 7, further including the step of washing the cells in a buffer containing potassium acetate, KCl, $MnCl_2 \cdot 4H_2O$, $CaCl_2 \cdot 2H_2O$, hexamine cobalt chloride and redistilled glycerol according to the procedure of Hanahan, J.Molec. Biol. 166, (1983), p.557.

9. The process according to any one of claims 1 to 8 wherein the step of freezing the cells comprises freezing the cells in a dry ice-ethanol bath.

10. The process according to any one of claims 1 to 9 wherein the step of harvesting the cells is done by centrifugation.

11. The process according to any one of claims 1 to 10, further including the step of storing the cells at -70°C after the freezing step.

12. The process according to any one of claims 1 to 11 wherein the step of growing the cells includes growing the cells in the presence of sodium chloride.

13. The process according to any one of claims 1 to 12 wherein the step of growing the cells includes growing the cells in the presence of sodium succinate.

14. The process according to any one of claims 1 to 13 wherein the growing step includes growing the cells at a temperature of at least 37°C and then growing the cells at a temperature of below 37°C.

15. The process according to any one of claims 1 to 14, further including the steps of thawing and refreezing the cells after said freezing step.

16. Competent transformable E.coli cells having a competency for plasmid DNA, and a transformation efficiency of at least $1 \times 10^9$ transformants per microgram of plasmid DNA.

**Revendications**

1. Procédé pour la production de cellules d'E. coli compétentes, aptes à être transformées, ledit procédé comprenant les étapes suivantes:
   croissance des cellules dans un milieu de croissance favorable à une température comprise entre 18°C et 32°C;
   récolte des cellules;
   remise rapide en suspension des cellules dans un tampon approprié; et
   congélation des cellules à approximativement -70°C.

2. Procédé selon la revendication 1, dans lequel les cellules d'E. coli ont une compétence pour l'ADN de plasmide.

3. Procédé selon l'une des revendications 1 ou 2, dans lequel les cellules d'E. coli sont d'au moins une des souches suivantes: RR1, HB101, DH1, DH5.

4. Procédé selon l'une quelconque des revendications 1 à 3, dans lequel le milieu de croissance favorable est un milieu complexe contenant du Mg.

5. Procédé selon l'une quelconque des revendications 1 à 4, dans lequel l'étape de croissance des cellules comprend la croissance des cellules pendant une période comprise entre 1 et 20 heures.

6. Procédé selon l'une quelconque des revendications 1 à 5, dans lequel l'étape de croissance des cellules comprend la croissance des cellules jusqu'à ce que la densité optique à 500 nm se situe dans l'intervalle de 0,5 à 1,0.

7. Procédé selon l'une quelconque des revendications 1 à 6, comprenant en outre l'étape de lavage des cellules dans un tampon avant l'étape de congélation.

8. Procédé selon l'une quelconque des revendications 1 à 7, comprenant en outre l'étape de lavage des cellules dans un tampon contenant de l'acétate de potassium, KCl, $MnCl_2.4H_2O$, $CaCl_2.2H_2O$, du chlorure de cobalt hexamine et du glycérol redistillé conformément au mode opératoire de Hanahan, J. Molec. Biol. 166, (1983), p.557.

9. Procédé selon l'une quelconque des revendications 1 à 8, dans lequel l'étape de congélation des cellules comprend la congélation des cellules dans un bain de carboglace-éthanol.

10. Procédé selon l'une quelconque des revendications 1 à 9, dans lequel l'étape de récolte des cellules est effectuée par centrifugation.

11. Procédé selon l'une quelconque des revendications 1 à 10, comprenant en outre l'étape de stockage des cellules à -70°C après l'étape de congélation.

12. Procédé selon l'une quelconque des revendications 1 à 11 dans lequel l'étape de croissance des cellules comprend la croissance des cellules en présence de chlorure de sodium.

13. Procédé selon l'une quelconque des revendications 1 à 12, dans lequel l'étape de croissance des cellules comprend la croissance des cellules en présence de succinate de sodium.

14. Procédé selon l'une quelconque des revendications 1 à 13, dans lequel l'étape de croissance comprend la croissance des cellules à une température d'au moins 37°C et ensuite la croissance des cellules à une température inférieure à 37°C.

15. Procédé selon l'une quelconque des revendications 1 à 14, comprenant en outre les étapes de décongélation et de recongélation des cellules après ladite étape de congélation.

16. Cellules d'E. coli compétentes, aptes à être transformées, ayant une compétence pour l'ADN de plasmide et un pouvoir de transformation d'au moins 1 x $10^9$ transformants par microgramme d'ADN de plasmide.

**Patentansprüche**

1. Verfahren zur Herstellung transformierbarer, kompetenter E.coli-Zellen, das folgende Schritte umfaßt:
   die Kultivierung der Zellen in einem wachstumsfördernden Medium bei einer Temperatur im Bereich von 18°C bis 32°C;
   das Ernten der Zellen;
   das schnelle Resuspendieren der Zellen in einem geeigneten Puffer;
   und das Einfrieren der Zellen bei ungefähr -70°C.

2. Verfahren nach Anspruch 1, in dem die E. coli-Zellen für Plasmid-DNA kompetent sind.

3. Verfahren nach einem der Ansprüche 1 oder 2, in dem die E. coli-Zellen mindestens einer der folgenden Stämme sind: RR1, HB101, DH1, DH5.

**4.** Verfahren nach einem der Ansprüche 1 bis 3, in dem das wachstumsfördernde Medium ein Mg-enthaltendes komplexes Medium ist.

**5.** Verfahren nach einem der Ansprüche 1 bis 4, in dem der Kultivierungsschritt der Zellen die Kultivierung der Zellen für einen Zeitraum innerhalb eines Bereiches von 1 bis 20 Stunden umfaßt.

**6.** Verfahren nach einem der Ansprüche 1 bis 5, in dem der Kultivierungsschritt der Zellen die Kultivierung der Zellen umfaßt, bis die optische Dichte bei 550 nm den Bereich von 0,5 bis 1,0 erreicht.

**7.** Verfahren nach einem der Ansprüche 1 bis 6, ferner enthaltend den Schritt des Waschens der Zellen in einem Puffer vor dem Schritt des Einfrierens.

**8.** Verfahren nach einem der Ansprüche 1 bis 7, ferner enthaltend den Schritt des Waschens der Zellen in einem Puffer, der Kaliumacetat, KCl, $MnCl_2 \cdot 4H_2O$, $CaCl_2 \cdot 2H_2O$, Hexaminkobaltchlorid und redestilliertes Glycerol nach dem Verfahren von Hanahan, J. Molec. Biol. 166, (1983), S. 557 enthält.

**9.** Verfahren nach einem der Ansprüche 1 bis 8, in dem der Schritt des Einfrierens der Zellen das Einfrieren der Zellen in einem Trockeneis-Ethanolbad umfaßt.

**10.** Verfahren nach einem der Ansprüche 1 bis 9, in dem der Schritt des Erntens der Zellen durch Zentrifugation ausgeführt wird.

**11.** Verfahren nach einem der Ansprüche 1 bis 10, ferner enthaltend den Schritt der Aufbewahrung der Zellen bei -70°C nach dem Schritt des Einfrierens.

**12.** Verfahren nach einem der Ansprüche 1 bis 11, in dem der Kultivierungsschritt der Zellen die Kultivierung der Zellen in Gegenwart von Natriumchlorid umfaßt.

**13.** Verfahren nach einem der Ansprüche 1 bis 12, in dem der Kultivierungsschritt der Zellen die Kultivierung der Zellen in Gegenwart von Natriumsuccinat umfaßt.

**14.** Verfahren nach einem der Ansprüche 1 bis 13, in dem der Kultivierungsschritt der Zellen die Kultivierung der Zellen bei einer Temperatur von mindestens 37°C und dann die Kultivierung der Zellen bei einer Temperatur unter 37°C umfaßt.

**15.** Verfahren nach einem der Ansprüche 1 bis 14, ferner umfassend die Schritte des Auftauens und Wiedereinfrierens der Zellen nach dem Schritt des Einfrierens.

**16.** Kompetente, transformierbare E. coli-Zellen, die für Plasmid-DNA kompetent sind und eine Transformationseffizienz von mindestens $\overline{1 \times 10^9}$ Transformanten pro $\mu$g Plasmid-DNA aufweisen.